# EUROPÄISCHE PATENTSCHRIFT

(11) **EP 2 001 858 B1**
(45) Veröffentlichungstag und Bekanntmachung des Hinweises auf die Patenterteilung: **27.07.2016**
(21) Anmeldenummer: 07723527.3
(22) Anmeldetag: 23.03.2007
(51) Int. Cl.: C07D 301/12, C07D 301/03

(54) **VERFAHREN ZUR HERSTELLUNG VON ALKYLENOXIDEN**
PROCESS FOR PREPARING ALKYLENE OXIDES
PROCEDE DE FABRICATION D'OXYDES D'ALKYLENE

(30) Priorität: 01.04.2006 DE 102006015268
(43) Veröffentlichungstag der Anmeldung: 17.12.2008
(73) Patentinhaber: Cognis IP Management GmbH, 40589 Dusseldorf (DE)
(72) Erfinder: GUTSCHE, Bernhard, 40724 Hilden (DE); FABRY, Bernd, 41352 Korschenbroich (DE); FRANZEN, Stefan, 59174 Kamen (DE)
(86) Internationale Anmeldenummer: PCT/EP2007/002576
(87) Internationale Veröffentlichungsnummer: WO 2007/112866

(56) Entgegenhaltungen:
- WO-A-2004/091771
- WO-A-2006/020709
- WO-A-2006/042598
- US-A1- 2002 028 164
- MARKOWZ, GEORG ET AL.: CHEMIE INGENIEUR TECHNIK, Bd. 76, Nr. 5, 2004, Seiten 620-625, XP002446823
- WAN: "1-PENTENE EPOXIDATION IN TITANIUM SILICALITE-1 MICROCHANNEL REACTOR.Experiments and Modelling" CHEMICAL ENGINEERING RESEARCH AND DESIGN, PART A, INSTITUTION OF CHEMICAL ENGINEERS, XX, Bd. 81, Nr. A7, August 2003 (2003-08), Seiten 753-759, XP008081909 ISSN: 0263-8762
- BERNDT, TORSTEN ET AL.: IND. ENG. CHEM. RES., Bd. 44, 2005, Seiten 645-650, XP002446824
- DATABASE WPI DERWENT PUBLICATIONS LTD., LONDON, GB; XP002446969 Database accession no. 2004-750806 & JP 2004 285001 A (SUMITOMO CHEM. CO. LTD.) 14. Oktober 2004 (2004-10-14) in der Anmeldung erwähnt

## Beschreibung

### Gebiet der Erfindung

Die Erfindung befindet sich auf dem Gebiet der Petrochemie und betrifft ein verbessertes Verfahren zur Herstellung von Ethylenoxid bzw. Propylenoxid unter Einsatz von strukturierten Mikroreaktoren.

### Stand der Technik

Alkylenoxide, wie Ethylen- und Propylenoxid, zählen zu den wichtigsten erdölbasierten Industriechemikalien. Ethylenoxid (EO) ist insbesondere Ausgangsprodukt für die Herstellung von Methylenglykol, das als Frostschutzmittel beispielsweise dem Flugbenzin zugesetzt wird. Da Ethylen- und Propylenoxid zudem auch mit allen Arten von Stoffen, die über azide Wasserstoffatome verfügen, abreagieren, eignet sie sich beispielsweise zur Anlagerung an Alkohole oder Amine unter Bildung von Polyalkylenglykolketten, die diesen Stoffen einen hydrophilen Charakter verleihen. Der wesentliche Auslass für diese Art von Verbindungen sind nichtionische Tenside, die insbesondere in Waschmitteln und Kosmetika Anwendung finden. Die Umsetzung von Ethylen- oder Propylenoxid mit Ammoniak liefert Alkanolamide, mit Kohlendioxid wird Ethylen- bzw. Propylencarbonat erhalten, die ebenfalls interessante Zwischenprodukte für die technische Chemie darstellen.

Ethylen- und Propylenoxid werden heute beinahe ausschließlich durch Direktoxidation der entsprechenden Alkylene an Silberkatalysatoren hergestellt:

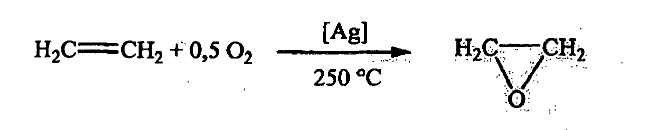

Die Reaktion ist beispielsweise für Ethylenoxid mit 120 kJ/mol exotherm und steht mit der vollständigen Verbrennung des Ethylens zu Kohlendioxid in Konkurrenz, die mit mehr als 1300 kJ/mol wesentlich stärker exotherm verläuft. Die technische Herstellung des Ethylenoxids erfolgt beispielsweise in der Regel in Rohrbündelreaktoren, die bis zu 1000 einzelne Röhren enthalten können und von außen mit einem flüssigen Wärmeträger, wie z.B. Tetralin gekühlt werden, um auch bei sich verstärkender Totaloxidation die Oxidationstemperatur von 230 bis 270 °C einhalten zu können. Der Katalysator, beispielsweise 15 Gew.-% Silber auf Al₂O₃, befindet sich als Schüttung in den Röhren. In der Regel wird der Oxidation mit Sauerstoff der Vorzug gegeben. Nichtsdestotrotz begrenzt man den Umsatz an Ethylen auf etwa 10 bis 15 %, da nur so Selektivitäten von maximal 75 bis 80 % erreicht werden können. Rund ein Viertel des teuren Einsatzmaterials wird also auf diese Weise zu Kohlendioxid verbrannt. Hinzu kommt, dass sich im Endprodukt typisch bis zu 2,5 Vol.-% Wasser und bis zu 10 Vol.-% Kohlendioxid befinden, die vor der Weiterverwertung mit hohem technischem Aufwand abgetrennt werden müssen. Die Probleme bei der Herstellung von Propylenoxid sind vergleichbar.

**Markowz et al.** (Markowz, G. et al., Chemie Ingenieur Technik, Bd. 76, Nr. 5, 2004, Seiten 620-625) beschreiben die Herstellung von Propylenoxid durch Reaktion von Propylen mit Wasserstoffperoxid in einem Mikroreaktor.

**Wan et al.** (Wan, Y.S.S. et al., Chemical Engineering Research and Design, Part A, Bd. 81, Nr. A7, August 2003, Seiten 753-759) befassen sich mit der Epoxidation von 1-Penten in einem Titanium-Silicalit-Mikrokanalreaktor.

Die WO 2004/091771 A betrifft einen Mikroreaktor zur Durchführung heterogenkatalysierter Reaktionen, welcher eine Vielzahl von vertikal oder horizontal und im Wesentlichen parallel angeordneter Räume aufweist. Beschrieben wird die Herstellung von Propylenoxid durch Reaktion von Propylen mit Wasserstoffperoxid in diesem Mikroreaktor.

Die WO 2006/020709 A beschreibt ein Verfahren zur Umwandlung von Ethylen in Ethylenoxid, bei welchem in einem Mikrokanalreaktor gearbeitet wird.

Die US 2002/028164 A1 befasst sich mit der Herstellung von Ethylen- oder Propylenoxid aus dem entsprechenden Alken durch Reaktion mit Wasserstoffperoxid in einem Reaktor, der eine Vielzahl von einander getrennter, paralleler, schlitzförmiger Reaktionsbereiche aufweist.
Berndt et al. (Berndt, T. et al., Ind. Eng. Chem. Res., Bd. 44, 2005, Seiten 645-650) beschreiben die Gasphasen-Epoxidation von Propylen und Ethylen.

Die WO 2006/042598 A beschreibt die Darstellung von Propylenoxid aus Propen und Wasserstoffperoxid in einem Mikroreaktor.

Aus einem Aufsatz von Schüth et al. in Ind. Eng. Chem. Res 41, 701-719 (2002) ist der Einsatz von Mikroreaktoren zur silberkatalysierten Oxidation von Ethylen zu Ethylenoxid bekannt. Als Oxidationsmittel wird hierbei reiner Sauerstoff verwendet. Aus der deutschen Patentschrift DE 10257239 B3 (ACA) ist ein Verfahren bekannt, bei dem man Olefine, beispielsweise auch Ethylen in Gegenwart eines Photosensibilisators oxidiert, wobei die Reaktion in einer Vielzahl parallel zu einander verlaufender Mikrofallfilmreaktoren erfolgt. Als Oxidationsmittel wird auch hier Sauerstoff verwendet. In der internationalen Patentanmeldung WO 01/083466 A1 (Merck) wird ein Verfahren zur Epoxidation von funktionalisierten Olefinen vorgeschlagen, bei der die Reaktion zwar ebenfalls in Mikroreaktoren, jedoch bei milden Bedingungen in der Flüssigphase erfolgt. Die Reaktion wird zudem ohne Katalysatoren durchgeführt. Ein ähnliches Verfahren zur Herstellung von pharmazeutischen Wirkstoffen ist aus der Japanischen Patentanmeldung JP 2004-285001 A2 (Sumitomo) bekannt: hier wird vorgeschlagen ungesättigte Einsatzstoffe wie beispielsweise 3-Dimethyl-2-(2-Methyl-1-propenyl)-cyclopropan-carbonsäuremethylester in der Flüssigphase mit Ozon umzusetzen, wobei die Reaktion in einem Mikroreaktor stattfinden kann; die Oxidation von Ethylen oder Propylen wird hier jedoch nicht erwähnt.

Die Aufgabe der vorliegenden Erfindung hat darin bestanden, ein Verfahren zur industriellen Herstellung von Ethylen- bzw. Propylenoxid zur Verfügung zu stellen, das frei von den Nachteilen des Stands der Technik ist und insbesondere bei hohen Umsätzen verbesserte Selektivitäten sowie Raum-Zeit-Ausbeuten liefert und die unerwünschte Totaloxidation der Einsatzstoffe so weit als möglich unterdrückt.

### Beschreibung der Erfindung

Gegenstand der Erfindung ist ein Verfahren zur Herstellung von Ethylenoxid und/oder Propylenoxid durch Inkontaktbringen von Ethylen und/oder Propylen mit einem Oxidationsmittel, welches sich dadurch auszeichnet, dass
(i) die Reaktion in einem Mikroreaktionssystem (µ-Reaktor) stattfindet und
(ii) als Oxidationsmittel eine Peroxoverbindung dient, wobei man als Peroxoverbindung Ozon einsetzt und wobei die Reaktion katalysatorfrei durchgeführt wird, wobei das Mikroreaktionssystem auf einem Träger aufgebracht ist, welcher einen Silizium-Glas-Verbund darstellt.

Überraschenderweise wurde gefunden, dass sowohl die Umsetzung von Ethylen als auch Propylen sowie Gemischen der beiden Gase mit Ozon unabhängig von den Explosionsgrenzen durchgeführt werden kann. Bei Einsatz von Ozon kann mit der technisch möglichen Maximalkonzentration gearbeitet werden. Durch das erfindungsgemäße Verfahren wird nicht nur die Selektivität und die Raum-Zeit-Ausbeute verbessert, was die Herstellung der Zielprodukte wesentlich ökonomischer macht, es wird gleichzeitig auch die Totaloxidation weitgehend vermieden. Dies führt nicht nur zu einer Einsparung an Rohstoffen, das Reaktionsendprodukt ist zudem auch praktisch nebenproduktfrei, so dass auf die ansonsten obligatorische und technisch aufwendige Trocknung und CO₂-Abtrennung verzichtet werden kann. Gleichzeitig erlaubt das Verfahren, einen Sauerstoffkreislauf zu fahren. Ein weiterer Vorteil besteht schließlich darin, dass die Reaktion katalysatorfrei durchgeführt wird.

### Strukturierte Reaktoren und Mikroreaktionssysteme

Ein zentrales Element der vorliegenden Erfindung besteht in der Erkenntnis, dass strukturierte Reaktoren es ermöglichen, die Oxidation von Ethylen und Propylen unabhängig von den Explosionsgrenzen durchzuführen, da die Reaktion isotherm geführt werden kann, die Reaktionspartner nur eine minimale Verweilzeit im Reaktor haben und die Reaktionskanäle Durchmesser aufweisen, die die Grenzspaltweite nicht übersteigen. Unter dem Begriff Grenzspaltweite ist dabei der maximale Durchmesser eines Reaktors zu verstehen, bei dem eine durch Explosion entstehende Flamme noch automatisch erlischt. Diese Umstände machen es möglich, beliebige Mischungen von Ethylen bzw. Propylen und Ozon zu verwenden und trotzdem den Reaktor auch sicher im Explosionsbereich zu betreiben.

Unter dem Begriff "strukturierter Reaktor" ist eine Anordnung von Reaktionskanälen zu verstehen, die einzeln, in Modulen oder auch alle gemeinsam betrieben werden können und sich in einer Matrix befinden, die zur Stabilisierung, Sicherung, Heizung oder Kühlung dient.

Die erfindungsgemäße Ausführungsform eines strukturierten Reaktors stellen Mikroreaktionssysteme dar, die auch allgemein als Mikro- oder µ-Reaktoren bezeichnet werden. Ihr Kennzeichen ist es, dass mindestens eine der drei Raumdimensionen des Reaktionsraumes eine Abmessung im Bereich von 1 bis 2000 µm aufweist und die sich damit durch eine hohe übertragungsspezifische innere Oberfläche, kurze Verweilzeiten der Reaktionspartner und hohe spezifische Wärme- und Stofftransportleistungen auszeichnen. Eine ausführliche Darstellung zu diesem Thema findet sich beispielsweise von Jähnisch et al. in Angewandte Chemie Vol. 116, 410-451 (2004**).** Beispielhaft sei auf die europäische Patentanmeldung EP 0903174 A1 (Bayer) verwiesen, in der die Flüssigphasenoxidation organischer Verbindungen in einem Mikroreaktor, bestehend aus einer Schar von parallelen Reaktionskanälen, beschrieben wird. Mikroreaktoren können dabei zusätzlich mikroelektronische Komponenten als integrale Bestandteile enthalten. Im Unterschied zu bekannten mikroanalytischen Systemen besteht bei den Mikroreaktoren keineswegs die Notwendigkeit, dass alle lateralen Dimensionen des Reaktionsraumes im µm-Bereich liegen. Vielmehr werden dessen Abmessungen ausschließlich durch die Art der Reaktion bestimmt. Dementsprechend kommen für bestimmte Reaktionen auch solche Mikroreaktoren in Frage, bei denen eine gewisse Zahl von Mikrokanälen gebündelt wird, so dass Mikro- und Makrokanäle bzw. paralleler Betrieb einer Vielzahl von Mikrokanälen nebeneinander vorliegen können. Vorzugsweise sind die Kanäle parallel zueinander angeordnet, um einen hohen Durchsatz zu ermöglichen und den Druckverlust so gering wie möglich zu halten.

### • Träger

Die Träger, in denen die Struktur und Maße der Mikroreaktionssysteme vorgegeben sind, können Materialkombinationen wie z.B. Silizium-Silizium, Glas-Glas, Metall-Metall, Metall-Kunststoff, Kunststoff-Kunststoff oder Keramik-Keramik oder Kombinationen dieser Materialien darstellen. Die erfindungsgemäße Ausführungsform ist ein Silizium-Glas-Verbund. Die Strukturierung eines beispielsweise 100 bis 2000, vorzugsweise etwa 400 µm dicken Wafers erfolgt vorzugsweise mittels geeigneter Mikrostrukturierungs- bzw. Ätztechniken, z.B. dem reaktivem Ionen-Ätzen ("Reactive Ion Etching"), wodurch in Silizium dreidimensionale Strukturen unabhängig von der Kristallorientierung gefertigt werden können [vgl. James et al. in Sei. Am. 4, 248 (1993**)**]. In gleicher Weise kann auch Glas behandelt werden.

Auf diese Weise behandelte Wafer können 10 bis 1000, vorzugsweise 100 bis 500 und insbesondere 200 bis 300 parallel zueinander verlaufende Mikroreaktionssysteme aufweisen, die entweder parallel oder sequenziell angesteuert und betrieben werden können. Die Geometrie, also der zweidimensionale Verlauf der Kanäle kann dabei sehr unterschiedlich sein: in Frage kommen Geraden, Kurven, Winkel und dergleichen sowie Kombinationen dieser Formelemente. Auch müssen nicht alle Mikroreaktionssysteme die gleiche Geometrie aufweisen. Die Strukturen zeichnen sich dabei durch Abmessungen von 50 bis 1500, vorzugsweise 10 bis 1000 µm und senkrechte Wände aus, wobei die Tiefe der Kanäle 20 bis 1800 und vorzugsweise etwa 200 bis 500 µm beträgt. Die Querschnitte eines jeden Mikroreaktionsraumes, die quadratisch sein können aber nicht müssen, liegen in der Regel in der Größenordnung von 20 × 20 bis 1500 × 1500 und insbesondere 100 × 100 bis 300x300 µm², wie dies beispielsweise auch von Burns et al. in Trans IChemE 77(5), 206 (1999**)** als typisch angegeben wird. Zur Versorgung der Mikroreaktionsräume mit den Edukten wird der Wafer an den dafür vorgesehenen Stellen durchgeätzt.

Zum Abschluss wird der strukturierte Wafer durch ein geeignetes Verfahren, z. B. anodisches Bonden, mit einem weiteren Wafer, aus Glas, vorzugsweise Pyrex-Glas, verbunden und die einzelnen Strömungskanäle dicht zueinander verschlossen. Selbstverständlich sind abhängig vom Substratmaterial auch andere Aufbau- und Verbindungstechniken zur Realisierung dichter Strömungssysteme möglich, die sich dem Fachmann selbstverständlich erschließen, ohne dass dieser hierzu erfinderisch tätig werden muss.

### • Strukturierung der Mikroreaktoren

Die Mikroreaktionssysteme können sich in eine oder mehrere Mischzonen, eine oder mehrere Reaktionszonen, eine oder mehrere Misch- und Reaktionszonen, eine oder mehrere Heiz- und Kühlzonen oder beliebige Kombinationen davon gliedern. Vorzugsweise weisen sie drei Zonen, nämlich zwei Reaktions- und eine Kühlzone auf, wodurch insbesondere zwei- oder mehrstufige Reaktionen in flüssiger oder auch gasförmiger Phase effizient durchgeführt werden können. In der ersten Zone erfolgt dabei die Vermischung zweier Reaktionsteilnehmer sowie deren Reaktion, in der zweiten findet die Reaktion zwischen dem Produkt der ersten Zone und einem weiteren Edukt statt, während in der dritten der Abbruch der Reaktion durch Absenken der Temperatur bewirkt wird. Dabei ist es nicht zwingend erforderlich, die erste und die zweite Reaktionszone thermisch streng voneinander zu trennen. Wenn nämlich die Zugabe eines weiteren Reaktionspartners erforderlich ist oder anstelle eines Mischungspunktes mehrere gewünscht werden, kann dies über die Zone 1 hinaus auch noch in der Reaktionszone 2 stattfinden. Die Mikroreaktionssysteme können dabei sequentiell oder aber gleichzeitig, d.h. parallel mit jeweils definierten Eduktmengen betrieben werden und dabei gleiche oder unterschiedliche Geometrien aufweisen. Eine weitere Möglichkeit, wie sich die Mikroreaktionssysteme in ihrer Geometrie unterscheiden können, besteht im Mischungswinkel, unter dem die Edukte aufeinander treffen und der zwischen 15 und 270° und vorzugsweise 45 bis 180° liegen kann. Des weiteren ist es möglich, jede der drei Zonen unabhängig voneinander zu kühlen oder zu heizen bzw. die Temperatur innerhalb einer Zone beliebig zu variieren, wobei die Reaktionsräume in diesem Beispiel Kanäle darstellen, deren Länge pro Zone 10 bis 500 mm betragen kann.

### Oxidation von Alkylenen

Als Oxidationsmittel zur Hersellung von Ethylen- bzw. Propylenoxid aus den entsprechenden Alkenen dient im Sinne der vorliegenden Erfindung Ozon Zusätzlich können die beiden Reaktionspartner im stöchiometrischen molaren Verhältnis eingesetzt werden. Als Radikalbildner empfiehlt es sich beispielsweise NO₂ zuzusetzen. in besonderer Vorteil des Verfahrens besteht indes darin, durch isotherme Fahrweise und kurze Verweilzeiten beliebige Mischungen von Ethylen bzw. Propylen und Oxidationsmittel einzusetzen. Selbst im theoretischen Fall einer Explosion würden die Flammen zudem von selbst erlöschen, da die Dimensionierung der Mikroreaktionssysteme unterhalb der Grenzspaltweite liegt. Wird als Oxidationsmittel Ozon eingesetzt, so wird vorzugsweise bei der technisch erzielbaren maximalen Ozonkonzentration gearbeitet, d.h. bis zu 196 g/Nm⁻³, d.h. 12-13 Gew.-% bei Verwendung von reinem Sauerstoff bzw. 60 g/Nm⁻³ oder 4 - 5 Gew.-% bei Verwendung von Luftsauerstoff.

Es ist weiter möglich, Inertgase, wie z.B. Methan in Mengen bis zu 50 Vol.% zuzusetzen; bei Einsatz von Luft erübrigt sich dies durch die Anwesenheit des Stickstoffs. Die Reaktionstemperatur kann zwischen 100 und 300 °C liegen. Vorzugsweise läuft die Oxidation bei 180 bis 250 und insbesondere 190 bis 220°C ab. Wird ein gasförmiges Oxidationsmittel eingesetzt, so kann dieses dem Ethylen bzw. Propylen direkt zugemischt werden; Die Reaktion kann im Bereich 0,1 bis 1 bar vorzugsweise 10 bis 100 mbar erfolgen. Höhere Drücke führen in der Regel zu geringerer Selektivität in Richtung der Zielprodukte.

### Aufarbeitung des Reaktionsgemisches

Nach Verlassen des Mikroreaktionssystems werden die einzelnen Produktströme zusammengeführt. Wie schon eingangs erläutert, besteht ein besonderer Vorteil des Verfahrens darin, dass die Totaloxidation weitgehend unterdrückt wird und infolge des geringen Wasser- und Kohlendioxidgehaltes für viele Anwendungen eine weitere Aufarbeitung nicht erforderlich ist.

Für den Fall, dass das Alkylenoxid substantiell frei von Wasser und CO₂ sein soll, kann die Aufarbeitung in an sich bekannter Weise erfolgen: dazu wird das Alkylenoxid zunächst in einem Absorber mit Wasser ausgewaschen, bevor es anschließend aus der wässrigen Lösung im Stripper abdestilliert wird. Dem nicht umgesetzten Restgas wird bis zur gewünschten Konzentration wieder Ethylen bzw. Propylen zugemischt. Anschließend wird es auf 5 bis 25 bar verdichtet, das enthaltene Kohlendioxid mit einer Waschlösung extrahiert und die gewünschte Konzentration des Oxidationsmittels eingestellt. Anschließend wird das Gasgemisch wieder in die Mikroreaktionssysteme zurückgeführt.

Beispiele 1, 2, 4, 5 und Vergleichsbeispiel 3

### Beispiele

Für die Untersuchungen wurde ein Mikroreaktionssystem bestehend aus einem 400 µm dicken Silizium-Wafer eingesetzt, der mit einem Pyrexglas-Wafer verbunden war. In den Silizium-wafer waren 20 parallele, linear verlaufende Kanäle mit einer Tiefe von 300 µm und einem Querschnitt der Mikroreaktionsräume von 300 x 300 µm² eingeätzt. Die Kanäle wurden parallel betrieben und waren jeweils zur Eduktaufgabe und zur Produktabnahme durchgeätzt. Untersucht wurde die Bildung von Ethylenoxid bzw. Propylenoxid durch Einwirkung von Ozon bzw. Wasserstoffperoxid ggf. in Gegenwart von geringen Mengen NO₂ als Radikalbildner auf Ethylen bzw. Propylen. Die Ergebnisse sind in Tabelle 1 zusammengefasst (Mittelwert aus 5 Messungen).

**Tabelle 1**

| **Versuchsergebnisse** | | | | | |
|---|---|---|---|---|---|
| **Paramater** | **1** | **2** | **3*** | **4** | **5** |
| c(Ethylen) [Vol.%] | 5 | 10 | 5 | - | - |
| c(Propylen) [Vol.-%] | - | - | - | 5 | 10 |
| Oxidationsmittel | O₃ | O₃ | H₂O₂ | O₃ | O₃ |
| c(Oxidationsmittel) [Vol.%] | 95 | 90 | 95 | 95 | 90 |
| Temperatur [**°**C] | 275 | 275 | 275 | 275 | 275 |
| Druck [mbar] | 100 | 100 | 100 | 100 | 100 |
| Verweilzeit [s] | 0,2 | 0,2 | 0,2 | 0,2 | 0,2 |
| Ethylenumsatz [%] | 10 | 8 | 9 | 20 | 18 |
| Selektivität zu EO/PO [%] | 95 | 92 | 94 | 93 | 91 |
| Selektivität zu CO₂ [%] | < 0,1 | <0,1 | < 0,1 | < 0,1 | < 0,1 |

| | | | | | |
|---|---|---|---|---|---|
| * 3 = Vergleichsbeispiel 3 | | | | | |

Wie die Beispiele zeigen, werden entsprechend dem erfindungsgemäßen Verfahren wesentlich höhere Selektivitäten erzielt als dies nach dem Stand der Technik bislang möglich war. Insbesondere wird auch die Totaloxidation beinahe völlig unterdrückt.

## Patentansprüche

1. Verfahren zur Herstellung von Ethylen- und/oder Propylenoxid durch Inkontaktbringen von Ethylen und/oder Propylen mit einem Oxidationsmittel, **dadurch gekennzeichnet, dass** man
(i) die Reaktion in einem Mikroreaktionssystem (µ-Reaktor) durchführt und
(ii) als Oxidationsmittel eine Peroxoverbindung einsetzt,
wobei man als Peroxoverbindung Ozon einsetzt und
wobei die Reaktion katalysatorfrei durchgeführt wird, wobei das Mikroreaktionssystem auf einem Träger aufgebracht ist, welcher einen Silizium-Glas-Verbund darstellt.

2. Verfahren nach Anspruch 1, **dadurch gekennzeichnet, dass** das Mikroreaktionssystem mindestens einen Zulauf für die Edukte und mindestens einen Ablauf für die Produkte aufweist.

3. Verfahren nach mindestens einem der Ansprüche 1 oder 2, **dadurch gekennzeichnet, dass** die Mikroreaktionssysteme durch geeignete Mikrostrukturierungstechniken auf den Träger aufgebracht werden.

4. Verfahren nach mindestens einem der Ansprüche 1 bis 3, **dadurch gekennzeichnet, dass** der Träger 10 bis 1000 zueinander parallel verlaufende Mikroreaktionssysteme aufweist, die sequentiell oder gleichzeitig mit den Edukten angesteuert werden können.

5. Verfahren nach mindestens einem der Ansprüche 1 bis 4, **dadurch gekennzeichnet, dass** die Mikroreaktionssysteme alle die gleiche oder unterschiedliche Geometrien aufweisen.

6. Verfahren nach mindestens einem der Ansprüche 1 bis 5, **dadurch gekennzeichnet, dass** die Mikroreaktionssysteme in mindestens einer Raumdimension Abmessungen im Bereich von 50 bis 1.500 µm aufweisen.

7. Verfahren nach mindestens einem der Ansprüche 1 bis 6, **dadurch gekennzeichnet, dass** die Mikroreaktionssysteme eine Tiefe von 20 bis 1.800 µm aufweisen.

8. Verfahren nach mindestens einem der Ansprüche 1 bis 7, **dadurch gekennzeichnet, dass** die Mikroreaktionssysteme Querschnitte von 20x20 bis 1.500x1.500 µm² aufweisen.

9. Verfahren nach mindestens einem der Ansprüche 1 bis 8, **dadurch gekennzeichnet, dass** die Mikroreaktionssysteme Kanäle darstellen, die eine Länge von 1 bis 500 mm aufweisen.

10. Verfahren nach mindestens einem der Ansprüche 1 bis 9, **dadurch gekennzeichnet, dass** die Mikroreaktionssysteme eine oder mehrere Mischzonen, eine oder mehrere Reaktionszone, eine oder mehrere Misch- und Reaktionszonen, eine oder mehrere Heiz- bzw. Kühlzonen oder beliebige Kombinationen davon aufweisen.

11. Verfahren nach mindestens einem der Ansprüche 1 bis 10, **dadurch gekennzeichnet, dass** man die Oxidation bei Temperaturen im Bereich von 100 bis 300 °C durchführt.

12. Verfahren nach mindestens einem der Ansprüche 1 bis 11, **dadurch gekennzeichnet, dass** man die Reaktion im Bereich von 0,1 bis 1 bar durchführt.

## Claims

1. A process for preparing ethylene oxide and/or propylene oxide by contacting ethylene and/or propylene with an oxidizing agent, wherein
(i) the reaction is performed in a microreaction system (µ-reactor) and
(ii) the oxidizing agent used is a peroxo compound,
wherein the peroxo compound used is ozone and
wherein the reaction is performed without catalysts, wherein the microreaction system is mounted on a support which is a silicon-glass composite.

2. The process according to claim 1, wherein the microreaction system has at least one inlet for the reactants and at least one outlet for the products.

3. The process according to at least one of claims 1 and 2, wherein the microreaction systems are applied to the support by suitable microstructuring techniques.

4. The process according to at least one of claims 1 to 3, wherein the support has from 10 to 1000 microreaction systems which run parallel to one another and can be sequentially or simultaneously actuated with the reactants.

5. The process according to at least one of claims 1 to 4, wherein the microreaction systems all have the same geometry or different geometries.

6. The process according to at least one of claims 1 to 5, wherein the microreaction systems have dimensions in the range from 50 to 1500 µm in at least one dimension.

7. The process according to at least one of claims 1 to 6, wherein the microreaction systems have a depth of from 20 to 1800 µm.

8. The process according to at least one of claims 1 to 7, wherein the microreaction systems have cross sections of from 20 × 20 to 1500 × 1500 µm².

9. The process according to at least one of claims 1 to 8, wherein the microreaction systems are channels which have a length of from 1 to 500 µm.

10. The process according to at least one of claims 1 to 9, wherein the microreaction systems have one or more mixing zones, one or more reaction zones, one or more mixing and reaction zones, one or more heating or cooling zones, or any combinations thereof.

11. The process according to at least one of claims 1 to 10, wherein the oxidation is performed at temperatures in the range from 100 to 300°C.

12. The process according to at least one of claims 1 to 11, wherein the reaction is performed in the range from 0.1 to 1 bar.

## Revendications

1. Procédé de fabrication d'oxyde d'éthylène et/ou de propylène par mise en contact d'éthylène et/ou de propylène avec un oxydant, **caractérisé en ce que**
(i) la réaction est réalisée dans un microsystème de réaction (réacteur µ) et
(ii) un composé peroxo est utilisé en tant qu'oxydant,
de l'ozone étant utilisée en tant que composé peroxo, et
la réaction étant réalisée dans catalyseur, le microsystème de réaction étant appliqué sur un support qui est un composite silicium-verre.

2. Procédé selon la revendication 1, **caractérisé en ce que** le microsystème de réaction comprend au moins une entrée pour les réactifs et au moins une sortie pour les produits.

3. Procédé selon au moins l'une quelconque des revendications 1 ou 2, **caractérisé en ce que** les microsystèmes de réaction sont appliqués sur le support par des techniques de microstructuration appropriées.

4. Procédé selon au moins l'une quelconque des revendications 1 à 3, **caractérisé en ce que** le support comprend 10 à 1 000 microsystèmes de réaction parallèles les uns des autres, qui peuvent être actionnés séquentiellement ou simultanément avec les réactifs.

5. Procédé selon au moins l'une quelconque des revendications 1 à 4, **caractérisé en ce que** les microsystèmes de réaction présentent tous la même géométrie ou des géométries différentes.

6. Procédé selon au moins l'une quelconque des revendications 1 à 5, **caractérisé en ce que** les microsystèmes de réaction présentent dans au moins une dimension de l'espace des dimensions dans la plage allant de 50 à 1 500 µm.

7. Procédé selon au moins l'une quelconque des revendications 1 à 6, **caractérisé en ce que** les microsystèmes de réaction présentent une profondeur de 20 à 1 800 µm.

8. Procédé selon au moins l'une quelconque des revendications 1 à 7, **caractérisé en ce que** les microsystèmes de réaction présentent des sections transversales de 20x20 à 1 500x1 500 µm².

9. Procédé selon au moins l'une quelconque des revendications 1 à 8, **caractérisé en ce que** les microsystèmes de réaction sont des canaux qui présentent une longueur de 1 à 500 mm.

10. Procédé selon au moins l'une quelconque des revendications 1 à 9, **caractérisé en ce que** les microsystèmes de réaction comprennent une ou plusieurs zones de mélange, une ou plusieurs zones de réaction, une ou plusieurs zones de mélange et de réaction, une ou plusieurs zones de chauffage ou de refroidissement, ou des combinaisons quelconques de celles-ci.

11. Procédé selon au moins l'une quelconque des revendications 1 à 10, **caractérisé en ce que** l'oxydation est réalisée à des températures dans la plage allant de 100 à 300 °C.

12. Procédé selon au moins l'une quelconque des revendications 1 à 11, **caractérisé en ce que** la réaction est réalisée dans la plage allant de 0,1 à 1 bar.
